(19)

Europäisches Patentamt
European Patent Office
Office européen des brevets

(11) **EP 3 095 380 A2**

(12) **EUROPEAN PATENT APPLICATION**

(43) Date of publication:
**23.11.2016 Bulletin 2016/47**

(51) Int Cl.:
*A61B 5/00* (2006.01)        *A61B 5/021* (2006.01)
*A61B 5/022* (2006.01)      *A61B 5/0295* (2006.01)
*A61B 5/053* (2006.01)      *A61B 5/11* (2006.01)
*G01G 19/50* (2006.01)

(21) Application number: **16167797.6**

(22) Date of filing: **29.04.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **30.04.2015 US 201514701054**

(71) Applicant: **Withings
92130 Issy les Moulineaux (FR)**

(72) Inventors:
• **CAMPO, David**
  **75015 Paris (FR)**
• **BUARD, Nadine**
  **92190 Meudon (FR)**
• **KARAM, Ghaleb**
  **92130 Issy les Moulineaux (FR)**
• **CARREEL, Eric**
  **92190 Meudon (FR)**

(74) Representative: **Cabinet Plasseraud
66, rue de la Chaussée d'Antin
75440 Paris Cedex 09 (FR)**

(54) **WEIGHING SCALE WITH EXTENDED FUNCTIONS**

(57)      Methods of determination of the blood pressure, determination of a heart stroke volume, determination of the state of stress or relaxation of a user, with use of a ballistocardiogram signal (21) reflecting the user's heart beats, with a measure of characteristic amplitude of ballistocardiogram signal, and measure for each couple of consecutive heart beats, beat time intervals DeltaHB(i) between successive heart beats, extracted from a ballistocardiogram signal (21) or from an impedance plethysmography signal measured at the user's foot, leading to determination of a heart rate variability index, over at least six successive heart beats, , leading to determination of mean arterial pressure, leading to determination of state of relaxation or stress of the user.

**FIG. 11**

**Description**

**FIELD OF THE INVENTION**

The present invention relates to weighing scale with extended functions, especially scales that provide, additionally to weight, information about some cardiovascular parameters.

**BACKGROUND OF THE DISCLOSURE**

In the known art, it is known from US8639226 [ to Withings] to measure **a body fat percentage** of a user standing barefoot on a scale. Besides, it is known from WO2014106716 [to Withings] to determine the **heart rate** of a user standing on a scale, by weight variations and foot-to-foot impedance analysis.

There is a need to provide from such a scale more information about health and physiological parameters of the user. There have been attempts to provide information about cardiovascular system like a rating of the arterial stiffness, like from example in document US2013/310700 [to Stanford]. However, photoplethysmograpy is a technique which suffers shortcomings when applied to a sole of a foot. Indeed the skin is substantially thicker at this place than at other locations where photoplethysmograpy is currently used. Also, there are few arteries located close to the skin of the foot sole.

Therefore, there is still a need to bring new solutions to provide information about cardiovascular system like a rating of the arterial stiffness, like a determination of the blood pressure, a determination of a heart stroke volume, a determination of the state of stress or relaxation of a user.

**SUMMARY OF THE DISCLOSURE**

According to a **first** aspect of the present disclosure, it is disclosed a method to determine a blood arterial pressure of an individual user (U) in a system comprising a smartphone (2), a cuff pressure monitor device (6) and a personal electronic scale (1) having a top surface with conductive pads, the method comprising the steps of :

S1- measure a first mean arterial pressure (MAP1) of the individual user (U) with the help of the cuff pressure monitor device, at a first instant GT1,
S2- determine a first arterial pulse wave velocity value (PWV 1) of the individual user (U) standing on the personal electronic scale, at a second instant GT2, temporally close to the first instant GT1

/a/- acquiring weight variations, and extracting therefrom a ballistocardiogram (21) of the user's heart beat,
/b/- acquiring impedance plethysmography signals across one of the foot of the user, and extracting therefrom a blood pulse signal (22) at the foot,
/c/-calculating a time delay (DT) between the heart beat and the blood pulse signal arriving at the foot,
/d1/ deducing therefrom a value of the arterial pulse wave velocity of the user,

S3- determine a second arterial pulse wave velocity value (PWV2) in a similar manner as for step S2, at a third instant GT3,
S4- determine a second mean arterial pressure (MAP2) of the individual user (U) from the first mean blood pressure MAP1 and a function of PWV1 and PWV2, namely

$$MAP2 = MAP1 + Fcorr\,(PWV1, PWV2).$$

Thanks to these dispositions, the individual user is able to know his/her mean arterial pressure (for example shown in the display of the smartphone), just by standing on the scale, without the necessity to use frequently the cuff pressure monitor device.

For example, the user may measure its arterial pressure every month with the cuff pressure monitor device, and the user measures its weight every day with the bathroom scale, and obtains therefrom daily up-to-date values of its mean arterial pressure.

According to a **second** aspect of the present disclosure, it is disclosed a method to assess a stroke volume of the heart systolic contraction of an individual user (U) standing on a personal electronic scale (1), the method comprising :

/a/- acquire weight variations, and extracting therefrom a ballistocardiogram signal (21) reflecting the user's heart beats,

- identify, in the ballistocardiogram signal, for <u>at least one</u> heart beat **HB**(i) exhibiting a pulse-like wave signal **PW**(i), having a first and a second significant negative apexes **I,K** and a first and a second significant positive apexes **H,J,**
- identify, one or more characteristic value **WCV** from at least two of the first and second positive and negative apexes, H,I,J,K,
- assess a user's stroke volume **SV,** as a mathematical function of the one or more characteristic value WCV.

Thanks to these dispositions, the individual user is able to know his/her heart stroke volume, from which the cardiac output CO can be calculated from Heart Rate (HR), by CO = HR x SV.

In some exemplary embodiments, the one or more characteristic value **WCV** can be defined by an integration of absolute signal of a portion of the wave signal **PW**(i), and/or by measuring one or more peak-to-peak amplitudes (A1,A2,A3) of the wave signal **PW**(i), and extracting the stroke volume value SV therefrom.

According to an auxiliary aspect of the present disclosure, it is also disclosed a <u>method</u> to :

- determine user's Heart Rate (HR) and cardiac output CO, by CO = HR x SV (from second aspect above)
- determine a user's cardiovascular parameter known as peripheral resistance RP, by dividing the mean arterial pressure (from first aspect above) by the Cardiac output, namely RP = MAP1/ CO, or RP = MAP2/ CO.

Thanks to these dispositions, the individual user is able to know his/her peripheral resistance.

According to a **third** aspect of the present disclosure, it is disclosed a <u>method</u> to assess a state of stress and/or relaxation of an individual user (U) standing on a personal electronic scale (1), the method comprising :

/a/- acquire weight variations, and extracting therefrom a ballistocardiogram signal (21) reflecting the user's heart beats,

/**PA1**/- identify, in the ballistocardiogram signal (21), <u>for each</u> of a plurality of consecutive heart beats HB(i), a pulse wave **PW**(i),

/**PA2**/- measure, for each pulse wave **PW**(i), at least one characteristic amplitude **WA**(i) of the pulse wave **PW**(i),

- measure, for each couple of consecutive heart beats, beat time intervals DeltaHB(i) between successive heart beats, from the ballistocardiogram signal (21) <u>or</u> from an impedance plethysmography signal (22) measured at the user's foot,

/**PR1**/- determine a user expiration phase whenever characteristic amplitude **WA**(i) decreases and/or beat time intervals DeltaHB(i) increases,

/**PR2**/- determine a user inspiration phase whenever characteristic amplitude **WA**(i) increases and/or beat time intervals DeltaHB(i) decreases,

- assess a state of stress and/or relaxation of the user, as a function of synchronization index between the inspiration/expiration phases and the user heart beats.

Thanks to these dispositions, the individual user is able to know his/her state of stress and/or relaxation.

In some exemplary embodiments, the method may further comprise :

/**PR3**/ - reconstruct, from steps /PR1/ and /PR2/, a respiration cycle as a cosine-like function of time, with null phase at a time of switch between inspiration and expiration (T_ie) or at a time of switch between expiration and inspiration (T_ei), wherein the synchronization index is defined from an evolution over time of a phase difference DeltaPhi, which separates the null phase of the respiration cycle and the nearest heart beat HB(i).

In some exemplary embodiments, the characteristic amplitude **WA**(i) of the pulse wave **PW**(i) may be defined by the amplitude $A_{JK}i$, measured from the second positive apex **Ji** to the second negative apex **Ki** or the amplitude $A_{IJ}i$ measured from the first negative apex Ii to the second positive apex **Ji.**

According to a **fourth** aspect of the present disclosure, it is disclosed a <u>method</u> to assess a state of fatigue of an individual user (U) standing on a personal electronic scale (1), the method comprising :

- measure, for each couple of consecutive heart beats, beat time intervals DeltaHB(i) between successive heart beats, extracted from the ballistocardiogram signal (21) or from an impedance plethysmography signal (22) measured at the user's foot,

- determine a heart rate variability index **HRVI,** over at least six successive heart beats.

Thanks to these dispositions, the individual user is able to know his/her state of fatigue.

In some exemplary embodiments, the heart rate variability index **HRVI** can be expressed by : Max **[DeltaHB(i)] - Min [DeltaHB(i)],** where indicia i is ranging from 1 to **i0**, i0 being the number of monitored heart beats when the user is standing on the scale, **i0** being at least 6.

In some exemplary embodiments, **i0** may be defined such that at least one complete respiration cycle is recorded during **i0** heart beats, preferably more one complete respiration cycle are recorded, whereby the respiration cycle is retrieved by the following steps :

/**PA1**/- identify, in the ballistocardiogram signal (21), for each of a plurality of consecutive heart beats HB(i), a pulse wave **PW**(i),

/**PA2**/- measure, for each pulse wave **PW**(i), at least one characteristic amplitude **WA**(i) of the pulse wave **PW**(i),

/**PR1**/- determine a user expiration phase whenever characteristic amplitude WA(i) decreases and/or beat time intervals DeltaHB(i) increases,

/**PR2**/- determine a user inspiration phase whenever characteristic amplitude **WA**(i) increases and/or beat time intervals DeltaHB(i) decreases.

In some exemplary embodiments, the heart rate variability index **HRVI** can be expressed as a Root Mean Squared of the Successive Differences of **DeltaHB**(i) over several successive heart beats.

In various embodiments of the invention, one may possibly have recourse in addition to one and/or other of the arrangements stated in the dependent claims.

## BRIEF DESCRIPTION OF THE DRAWINGS

Other features and advantages of the invention appear from the following detailed description of one of its embodiments, given by way of non-limiting example, and with reference to the accompanying drawings, in which:

- Figure 1 illustrates a body of a user standing on a weighing scale according to the invention,
- Figure 2 is a closer side view showing one of the foot of the user,
- Figure 3 is a top schematic view of the weighing scale, the right half illustrating a first embodiment, and the left half illustrating an alternative embodiment,
- Figure 4 is a time chart showing various signals relating to the heart activity,
- Figure 5 illustrates an exemplary functional diagram of the scale
- Figure 6 illustrates a system comprising the scale for managing users profiles,
- Figure 7 illustrates details of a ballisto-cardiogram signal,
- Figure 8 illustrates a ballisto-cardiogram signal over a longer time, and influenced by the respiration,
- Figure 9 illustrates various detailed plethysmography signals,
- Figure 10 is similar to Fig 8 and shows a blood pulse obtained from an impedance plethysmography signal,
- Figure 11 illustrates diagrammatically a state chart of the process,
- Figure 12 is a time chart showing mean arterial pressure assessment.

## DETAILLED DESCRIPTION OF THE DISCLOSURE

In the figures, the same references denote identical or similar elements.

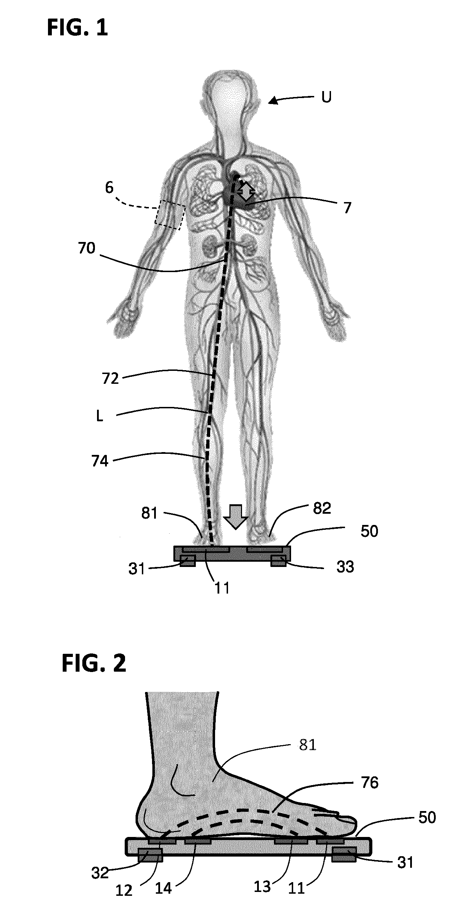

Figure 1 shows an individual **user U** standing on a weighing scale **1** (also often called 'bathroom scale'). The body of the user is shown translucent, the heart **7** produces a pressure pulse in the arterial network causing the user's blood to circulate in arteries toward lungs, head and all other organs, blood coming back to the heart via veins.

In particular, the left ventricular contraction periodically imparts a pressure pulse in the arteries responsible for the pulsatile movement blood in the arteries from the heart towards the other organs. More particularly, the pressure pulse and the blood move toward the feet **81,82** via the descending aorta **70,** the femoral artery **72,** and the tibial artery **74.**

Of particular interest for the following description, at each ventricular contraction, the pulsatile movement of blood in the arteries is accompanied by a recoil effect of the body which reflects into a small change in weight sensed by the weight sensors of the scale.

Besides, each ventricular contraction induces pressure pulse through the aorta **70** and the leg arteries **72,74** down to the feet. This pressure pulse sets in motion the blood in the arteries. When this pressure pulse arrives at the feet, the resulting change of volume of the blood in the feet arteries can be measured by the method known as impedancemetry.

The pressure pulse travels for a certain time from the heart to the feet. This travel time is somehow representative of the health state of the circulatory system of the user. More precisely, this travel time is representative of the arterial stiffness of the circulatory system of the user. The velocity of the blood pressure pulse is usually comprised between 5 m/s and 15 m/s.

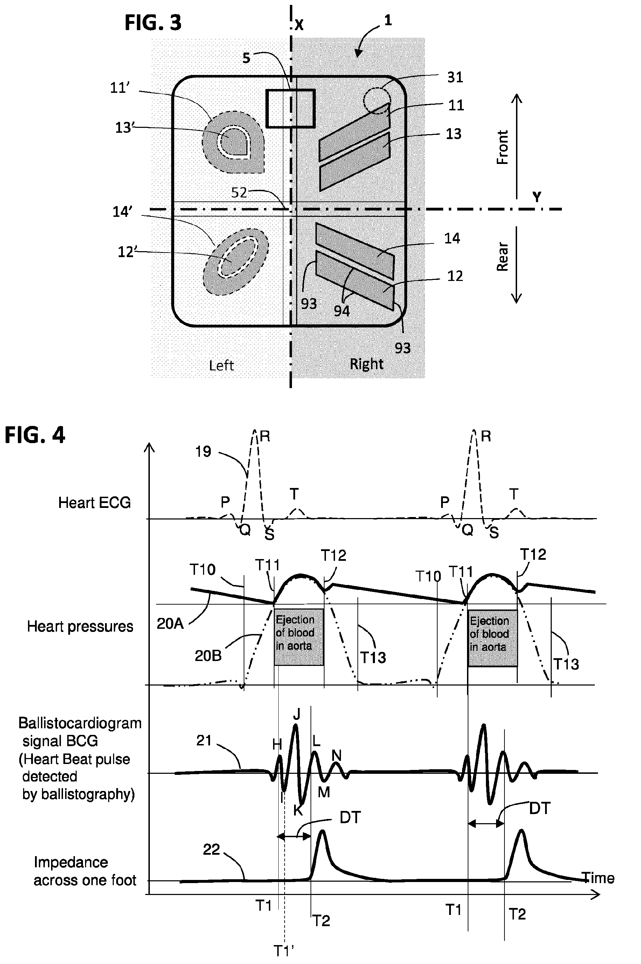

As shown in Figures 2, 3, 4 and 5, the scale has a controller **4,** a battery **8** and a display **5,** and comprises as known per se weight sensing element(s) **31,32,33,34,** for example four strain gauges as described in WO2014106716 the content of which is incorporated here by reference. The main function of the scale 1 is to determine the weight of a person standing on the scale. Also, the small variations over time of the sensed weight can be used to extract signals representative of certain physiological activity of the human body, in particular regarding the heart, this technique is called **ballistocardiography.** In particular, the heart beat activity reflects in small variations over time of the sensed weight, which are reflected in a ballistocardiogram (in short '**BCG**'), as shown at ref **21** in Fig 4. The extraction can be performed as explained with a comprehensive manner in WO2014106716. Shortly, the four strain gauges are arranged two by two, in two Wheastone bridges **35,36,** either in a right-left logic or in a front-rear logic.

Each Wheastone bridge ouputs a respective signal **78,79,** forwarded to the controller 4, where they enter into a sum-device and then further into an analog-to-digital converter or first into analog-to-digital converters and then further into a sum-device (not shown) to calculate the weight **W** therefrom, as known per se.

One solution, among others, to work out ballistogram signals, is to pick-up signals at the outputs of the Wheastone bridges **35,36,** enter them into band pass filters **37,38,** sum the resulting signals **dW1, dW2** in a sum-device **39** and input such signal **21** into the controller **4.**

Of course, it is possible, conversely, to perform summing before filtering, in order to issue a ballistogram signal **21.** This is referred to as step /**a**/ of the disclosed method.

It is not excluded to directly convert analog signals output by the Wheastone bridges **35,36** and perform all the subsequent treatments with digital operations within the controller. Band pass filters **37,38** can have the following cut off frequencies [0.5 Hz - 25 Hz], which discards continuous and low frequency components and also eliminates noise.

Further, the scale comprises, on its top surface **50,** at the right side of the scale, four conductive pads **11-14,** intended to come in contact with the right foot of a person standing on the scale. As drawn, right and left sides of the scale are separated by a medial sagittal axis **X,** and front and rear portions of the scale are separated by a medial transverse axis **Y.**

The user can stand preferably barefoot on the scale; however, even if the user bears socks, it does not prevent the disclosed method to operate properly.

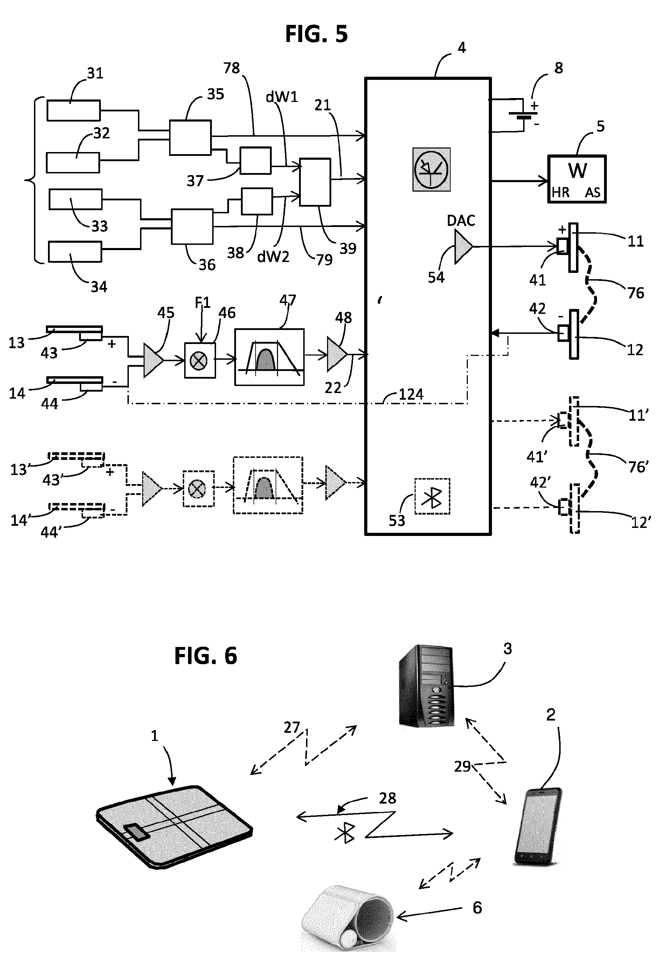

An electrical current is injected between a first pad **11** and a **second** pad **12,** and this current flows through the foot along path **76** inside the foot. This current is not harmful and not dangerous, it is limited in amps to less than **0,5 mA.** This current can be generated by a current source or a voltage source. The first conductive pad **11** is coupled to a first electrode **41** which is coupled to a current output of the scale, controlled by a current or voltage control signal of the controller **4** (via for instance a Digital Analog Converter 54, or another method (not shown), and adequate signal conditioning (not shown), cf. Fig 5). The first conductive pad **11** is located at a front portion of the top surface of the scale and is conventionally the place where current is entered into the foot of the user ('+' terminal).

The second pad **12** is coupled with a second electrode **42** which is coupled to a current input (also called 'current return') of the scale reference. The second pad **12** is located at a rear portion of the top surface of the scale and is conventionally the place where current comes out of the foot of the user ('-' terminal).

Advantageously the injected current is a sine alternating current. The applied frequency **F1** is in the range [10kHz-200kHz], preferably about 50kHz, such that the current injection is not harmful to the user and unnoticed by him. Preferably the injected current has a predefined fixed frequency F1 and a steady amplitude, and is generated by a current source or a voltage source.

Blood arriving in the foot produces a modulation (at the frequency of the heart rate) of the impedance. The amplitude of the modulation is rather small, it accounts for about 1/1000 of the impedance of the body (foot to foot).

Simultaneously with the current injection, a resulting voltage is measured across a third pad 13 and a fourth pad 14. Since the voltage is modulated at the same frequency as per the injected current, demodulation is required to extract the baseband frequency voltage exhibiting only the low frequency modulation induced by the blood volume variation, as detailed below.

The third pad **13** is coupled with a third electrode **43** which is coupled to a first voltage input of the controller. The third pad **13** is located at the front portion of the top surface of the scale, close to the first pad.

The fourth pad **14** is coupled with a fourth electrode **44** which is coupled to a second voltage input of the controller. Advantageously, a differential measuring technique is carried out.

The fourth pad **14** is located at the **rear** portion of the top surface of the scale close to the second pad.

As illustrated, the third pad 13 and the fourth pad 14 are interposed between the first pad 11 and the second pad 12; in other words, measured voltage is picked up inside the current injection area in the foot.

In an alternative reversed embodiment, the first pad **11** and the third pad **13** could be arranged at the rear portion (instead of front portion), and the second pad **12** and the fourth pad **14** could be are arranged at the front portion (instead of rear portion).

More precisely, as already mentioned, the periodic heart beat induces a small periodic blood volume variation in the foot; and since the blood volume variations in the foot results in corresponding electrical impedance, impedance variations are representative of the blood volume variations which are resulting in turn from the blood flow pulse arriving at the foot from the heart. This is also known as "impedance plethysmography" (**'IPG'** in short).

In other words, the scale controller **4** acquires impedance plethysmography signals across the foot of the user resulting from a blood flow pulse at the foot, in particular a variation of the impedance, resulting from a corresponding variation of the blood volume at the foot.

Therefore the IPG signal **22** will be the result of a demodulation of the voltage measured between pads 13 and 14, such demodulation being performed by a dedicated hardware block upfront the controller.

More precisely, with reference to Fig 5, circuit **45** is an amplifier which amplifies the voltage difference between electrodes **44** and **43.** Circuit **46** is an amplitude demodulator, to issue a baseband frequency signal. Circuit **47** is a band pass filter and circuit **48** is another amplifier to result in a ready-to-use impedance plethysmography signal **22.**

The thus demodulated and filtered analog voltage is digitally handled by the controller 4. This is referred to as step /**b**/ of the disclosed method.

The stages of the electronic chain can be exchanged. For instance demodulation can be done before amplification.

It is to be noted that the current input 12 and the second voltage input 14 are distinct and separate, as illustrated, to enhance accuracy and signal decoupling. However, in a variant embodiment, the current input 12 and the second voltage input 14 can be electrical-wise common (chain-dotted line **124** at figure 5). In another variant embodiment, not shown, the second and fourth pads **12,14** are formed as a single pad, such that only three conductive pads (instead of 4) are sufficient to measure the impedance of the foot. In another variant embodiment, not shown, current input 11 and the second voltage input 13 can be electrical-wise common. In another variant embodiment, not shown, the first and third pads **11, 13** are formed as a single pad.

The impedance plethysmography signal 22 resulting from the above described signal conditioning is shown at Fig 4, with other signals.

Signal **19** shows an indicative heart electrocardiogram (ECG) reflecting the heart electrical activity, as known per se.

Signals **20A** and **20B** show superposed respectively the ventricular (20B) and aortic (20A) pressures during cardiac cycles. The mechanical contraction of the heart causes the rise of the ventricular pressure. **T10** denotes the closing of the mitral valve, inducing the beginning of the pressure rise in ventricle (isovolumic contraction); at the instant **T11**, when the ventricular pressure 20B equals the diastolic pressure in the aorta, the aortic valve opens and blood is ejected from the ventricle into the aorta, this phase lasts until the instant **T12** when the ventricular pressure 20B becomes lower than the aortic pressure, with the closure of the aortic valve. **T13** denotes the return of the ventricle to an idle state.

Besides, **BCG** signal **21** shows the corresponding ballistocardiogram (responsive to heart beat), which exhibits a periodic occurrence of a pulse-like wave having negative apexes **I,K,M** and positive apexes **H,J,L,N.** Instant **T1** is defined to be the first positive apex **H.** Instant **T1'** is defined to be the first negative apex **I.** Either **T1** and **T1'** can be used to estimates of the opening of the aortic valve at **T11.** Alternately, other markers of the BCG could be used to estimate the opening of the aortic valve at **T11,** for instance the instant of the maximum of the time derivative of the BCG between H and I.

As discussed above, the impedance plethysmography signal 22 is responsive to an increase of the blood volume. Instant **T2** is defined to be the first detected significant rise in the signal. The time difference **T2-T1** is related to the pulse transit time (PTT) of the pressure pulse from the heart to the foot. We note **DT=T2-T1,** and this time delay calculation is referred to as step /**c**/ of the disclosed method.

**DT** can be the averaged result of three or more consecutive calculations, for more accuracy and/or reliability.

**DT** can typically be comprised between 50ms and 300ms, generally between 80ms and 200ms. For a normal young individual, the arteries are flexible, and the time delay **DT** is rather long, typically 120ms or more depending on his height. For a normal old individual, the arteries are more rigid, and the time delay **DT** is shorter, typically 110ms or less depending on his height. Of course these values are indicative only. Certain young individuals may have time delays shorter than 120ms, as well as certain old individuals may have time delays longer than 110ms.

On the display **5,** the user can read the weight **W,** the heart rate **HR** and a value of arterial stiffness **AS**. The arterial stiffness **AS** stands for the flexibility of arteries wall tissues. HR can be determined from the BCG signal 21 and/or from IPG signal 22.

One way to express Arterial Stiffness **AS** is to use the **pulse wave velocity (PWV)** of the pressure pulse. It is calculated as **PWV** = **f (L/DT)** with **f** being a linking function.

The path length **L** from the heart to the foot is calculated with a function of the height of the user. **DT,** as explained above is related to the pulse transit time of the blood pressure pulse. **PWV** can therefore be expressed in m/s. The PWV of the user can be compared to a normal range given the age and gender of the user and optionally also the blood pressure type. Another way to express Arterial Stiffness is as an arterial equivalent age, or an arterial range of age, reflecting the state of the arteries compared to a normal state given the chronological age and gender of the user. Therefore, the display 5 can write for example an interval **[23 y/o - 26 y/o].**

A value for the arterial stiffness can be given either at each measurement, or can be profitably averaged over several subsequent measurements to smooth out daily variations.

An arterial stiffness value found outside the expected range for an individual may denote some cardiovascular problem, an atherosclerosis or atheromatosis.

It is noted here that an image of the cardiovascular system compliance **C** can also be inferred from the above process; Compliance **C** is generally a ratio between arterial volume change and pressure change and is proportional to $1/PWV^2$ according to Moens-Korteweg equation known per se.

As illustrated in <u>figure 6</u>, the scale **1** is used preferably in a system comprising a smartphone **2** or the like and a remote server **3** (or cloud service).

The scale **1** and the smartphone **2** are able to be in communication through a wireless short-range communication link **28,** preferably Bluetooth™ **53** interface. However, instead of Bluetooth™, any wireless remote short-range communication link can be used.

As known per se, the smartphone **2** is able to be in communication through cellular wireless network **29** with generally speaking internet, and particularly the remote server **3** (or the cloud service). It is not excluded to have a direct link **27** from scale **1** to the remote server **3** (or cloud service).

Each individual which may use the scale can be defined at least by a user profile which comprises the height, the age and the gender of the individual. This data can be entered via the graphic tactile interface of the smartphone, and can be stored in the server 3.

Also, the scale **1** can recognize automatically which user is currently standing on it, thanks to US20140309541weight expected intervals, as taught in US8639226.

The height, the age and the gender and optionally also the blood pressure type of the individual are used to adjust the interpretation of the value of **DT** (or **PWV**) with regard to normally expected values, i.e. min-max normal interval for a particular type of individual.

The height, the age and the gender of each known individual can be sent from the smartphone **2** down to the scale **1,** for example, at the first use.

There may be provided abacus or regression curves in the server 3 to which the user measured values are compared. There may be provided individual storage with past measurements which constitutes a personal history data, stored either in the smartphone and/or in the server **3.**

The system can also comprise a cuff blood pressure monitor device 6, such a device is known for example from US20140309541. From time to time (typically every month or every fortnight), the user measures his/her blood pressure with the help of the cuff blood pressure monitor device **6.**

What is known under the term "blood pressure" or "arterial pressure" of an individual usually comprises two values : a systolic pressure $P_{syst}$ (higher value) and a diastolic pressure $P_{diast}$ (lower value); they may be expressed in the following units : kPa or mmHg.

Another value, known as "mean arterial pressure" (in short **MAP**) is defined from the two above mentioned values, obtained by the following equation:

$$\textbf{Mean Arterial Pressure} = 1/3 \ \textbf{P}_{\textbf{syst}} + 2/3 \ \textbf{P}_{\textbf{diast}}$$

$P_{syst}$, $P_{diast}$ (optionnaly together with the mean arterial pressure) can be displayed locally on a display of the cuff pressure monitor device **6** and/or sent to the smartphone **2** for storage and further data processing (personal history, ..).

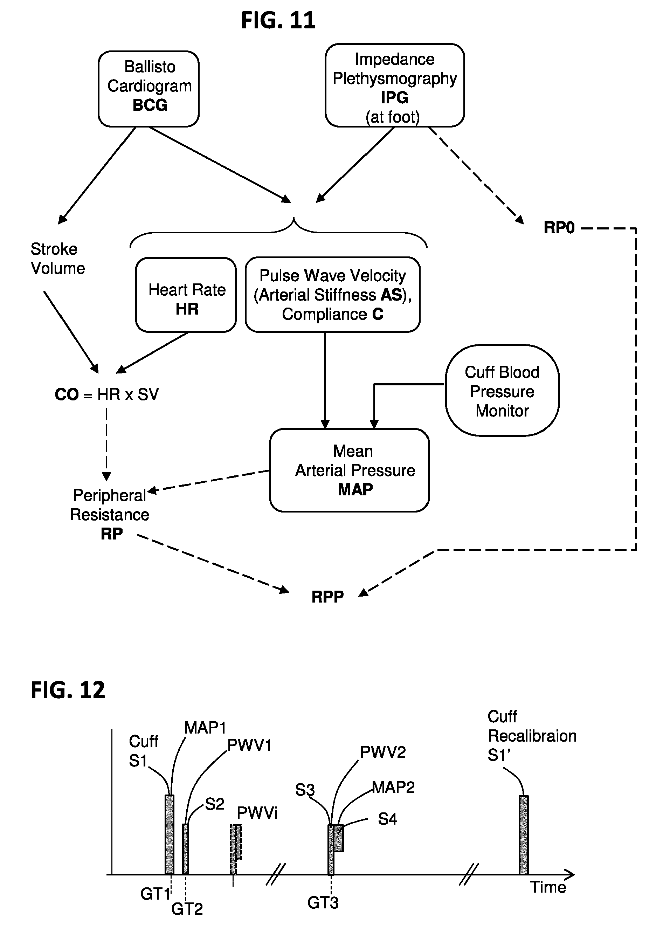

According to an aspect of the present disclosure, with reference to <u>Figure 12</u>, the user **U** measures a first mean arterial pressure **MAP1** with the help of the cuff pressure monitor device 6, at a first instant **GT1** (step denoted 'S1').

Approximately at the same time, just before or just after, at a second instant **GT2,** the user **U** stands on the scale 1, and **BCG** and **IPG** signals are analyzed as explained above, in particular the steps denoted /**a**/, /**b**/ and /**c**/. Then, at a step denoted /**d1**/, a first arterial pulse wave velocity value **PWV1** is deduced therefrom **PWV1=f(L/DT)** as explained above (step denoted 'S2').

Later, at a third instant **GT3,** i.e. later in the same day, or the next day, or still another day, the user again stands on the scale **1,** and, in a similar way as for **PWV1b**, a second arterial pulse wave velocity value **PWV2** is determined (step **'S3'** including steps /**a**/,/**b**/,/**c**/,/**d1**/).

Advantageously, after determination of this second arterial pulse wave velocity value **PWV2,** the disclosed method proposes, at step denoted '**S4**', to determine a second mean arterial pressure **MAP2** of the individual user **U** from the first mean blood pressure **MAP1** and a function of **PWV1** and **PWV2**, namely **MAP2 = MAP1 + Fcorr (PWV1, PWV2).**

**Fcorr** is the correction function. Fcorr gives a positive output if PWV2 is greater than PWV1 and a negative output if PWV2 is smaller than PWV1; Fcorr may rely on an abacus, or may be expressed as a function of PWV1 and PWV2. One possible expression of this function is **MAP2** = **MAP1** + **KZ** x (**PWV2 - PWV1),** where **KZ** is a parameter. This calculation is performed every time the user weights herself/himself, typically on a daily basis.

Indeed, it is known that an increase of blood pressure causes an increase of PWV, and a decrease of blood pressure causes a decrease of PWV.

In other words, the short term variations of blood pressure are determined through the change of pulse wave velocity, knowing that the arterial stiffness (flexibility of arteries wall) evolves very slowly over time.

The mean arterial pressure **MAP1** measured with the help of the cuff pressure monitor device 6 (baseline calibrated arterial pressure) can be used to adjust the arterial equivalent age of the user.

The second, short term, blood pressure data MAP2 can also be used to adjust the arterial equivalent age from the values of PWV. Further, MAP2 can be sent to the smartphone 2 and to the server to enhance the personal history.

Advantageously, successive measurements of the MAP can be averaged in order to smooth the short term variabitity of the PWV caused by the variations of blood pressure, thus making the measurement of the arterial compliance more accurate.

Recalibration of the baseline pressure with the cuff device 6 is necessary only when the PWV/average arterial stiffness changes significantly. The need to proceed to a measurement with the cuff blood pressure monitor 6 can be signaled to the user with a notification send via a relevant application on the smartphone 2.

Regarding size and shape of conductive pads **11-14,** in a preferred embodiment illustrated on the right side at figure 3, each pad can be a trapezoidal shape with two long sides (segments) **94** and two short sides **93,** the long sides extending substantially radially from the center portion **52** (where axis X and Y cross) of the top surface **50** of the scale.

On Figure 3 and Figure 5, there are shown in dotted line additional conductive pads **11',12',13',14',** which can be seen functionally as a duplicate of the already commented pads at the other side of the scale. Similarly, additional electrodes **41'-44'** are used to connect the additional conductive pads **11'-14'** to the internal electrical circuits of the scale 1.

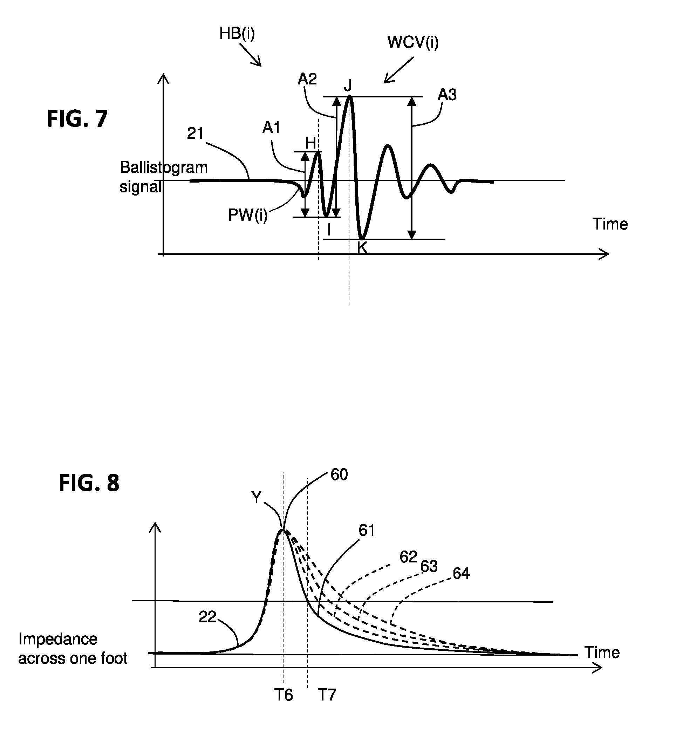

According to a further aspect of the disclosure, illustrated at <u>Figure 7</u>, which is independent from the impedance plethysmography signal analysis, there may be provided a further analysis of the ballistocardiogram signal **21**. More precisely, said signal exhibits a periodic occurrence of a pulse-like wave **PW**(i) having a first negative apex **I** and a second negative apex **K** and a first positive apex **H** and a second positive apex **J.**

The controller can measure a first amplitude **A1**, from the first positive apex **H** to the first negative apex **I,** a second amplitude **A2**, from the first negative apex **I** to the second positive apex **J,** a third amplitude **A3**, from the second positive apex **J** to the second negative apex **K**. The three resulting values of amplitudes **A1**, **A2**, **A3** are known to be related to various aspects of systole, for instance the force of ejection of the blood by the heart (the systolic ejection force), or the work of the heart at systole, or the volume of blood ejected at systole (the stroke volume). The three values **A1**, **A2**, **A3** can be called characteristic amplitudes **WA**. The three values **A1**, **A2**, **A3** are thus used to assess these quantities describing systole. For instance, the stroke volume is given by **SV** = **G** (**A1**,**A2**,**A3**), **G** being a linking function.

An example of the linking function **G** is can be given by :

$$\mathbf{G} = \mathrm{K}\ \mathrm{x}\ \sqrt{\alpha1A1\ +\ \alpha2A2\ +\ \alpha3A3}\ \mathrm{x}\ (\mathrm{HR})^{\mathrm{BR}}$$

where K, $\alpha1$, $\alpha2$, $\alpha3$ and BR are either predefined coefficients or parameters depending on user profile (age, gender, height, mean arterial pressure). **HR** is the user's heart rate.

More generally, it is possible to identify from the ballistocardiogram signal **21** one or more characteristic value **WCV** from at least two of the first and second positive and negative apexes, **H,I,J,K.**

Alternately, other quantities describing systole may be assessed using other specific values calculated from the ballistocardiogram pulse PW(i), for instance any integral of the absolute value of the signal between characteristic markers of systole (e.g. H,I,J and K).

For example, it can be chosen $\mathbf{WCV} = \int_{H}^{Q} |PW(i)(t)|dt,$ **Q** being either apex **I** or apex **J,** H being the first positive apex. **SV** is then inferred from such **WCV.**

Alternately it is possible to identify at least one characteristic amplitude **WA**(i) which can be defined from an auxiliary BCG signal. Such auxiliary **BCG** signal is obtained from base BCG signal **21** after filtering operations conveniently chosen to enhance certain features of the pulse wave **PW**(i).

With the stroke volume, the controller can further determine the Cardiac Output **CO,** such as **CO** = **HR x SV,** where **HR** is the heart rate which may be obtained, from BCG and/or IPG, or from known method as described in WO2014106716.

With the cardiac output and the mean blood pressure **MAP** obtained as described above, the controller can further determine a cardiovascular parameter known as **"peripheral resistance"** denoted **RP,** such as **RP** = **MAP** / **CO.**

For instance, with regard to calculations mentioned above :

$$RP = MAP1/CO, \text{ or } RP = MAP2/CO$$

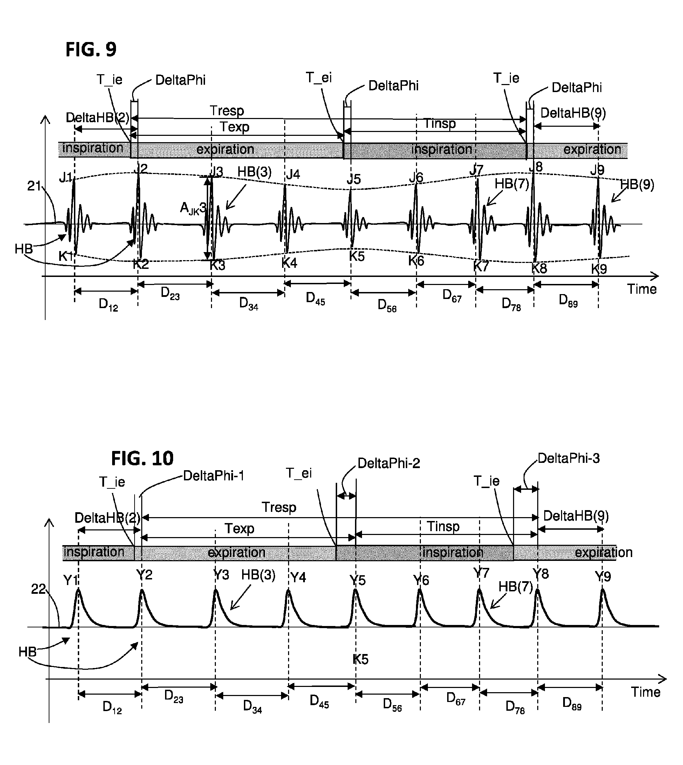

According to a further aspect, illustrated at <u>Figure 9</u>, which can be independent from the impedance plethysmography signal IPG analysis, the ballistocardiogram signal BCG **21** and its amplitudes is analyzed over a longer period, at least six heart beats in the illustrated case.

In the ballistocardiogram signal BCG **21**, each heart beat is denoted **HB(i)** and generates a corresponding pulse wave **PW**(i) as already mentioned.

There is defined characteristic amplitude **WA**(i) for each **PW**(i) which gives a plurality of consecutive characteristic amplitudes **WA**(i). Such series of **WA**(i) are compared from one beat to another, in order to retrieve a modulation caused by the respiration of the user standing on the scale.

In particular, **WA**(i) can be defined from the highest positive apex **J** and the deepest negative apex **K.** More precisely, WA(i) can be defined by the peak to peak amplitude $A_{JK}$ between points J and K. Notably as shown, there is provided an array of values J1-J9, K1-K9; $A_{JK}1$- $A_{JK}9$ which are analyzed to extract a low frequency amplitude modulation reflecting the respiration rate.

**WA**(i) can also be defined in another manner from an auxiliary BCG signal. Such auxiliary BCG signal is obtained from base BCG signal **21** after filtering operations conveniently chosen to enhance certain features of the pulse wave.

According to a further aspect, illustrated at <u>Figures 9 and 10</u>, the beat-to-beat time intervals are measured from the base ballistocardiogram BCG <u>or</u> from the impedance plethysmography signal 22 measured at the user's foot.

$$\textbf{DeltaHB(i)} = \text{Time Delay from HB (i-1) to HB (i), likewise denoted } \mathbf{D_{(i-1)(i)}} \text{ at Figures 9}$$

and 10.

Over time periods of a few seconds, beat-to-beat time intervals are known to be modulated by the respiration, which is known as respiration sinus arrythmia.

The time intervals **DeltaHB(i)** (shown as $D_{12}$ , $D_{23}$, ..., $D_{89}$) between successive **J** apexes on BCG signal 21 (respectively on successive **Y** apexes on impedance plethysmography signal 22) tend to be shorter during inspiration and longer during expiration.

Therefore, a user expiration phase is assumed whenever characteristic amplitude **WA**(i) decreases and/or beat time intervals DeltaHB(i) increases.

Similarly, a user inspiration phase is assumed whenever characteristic amplitude **WA**(i) increases and/or beat time intervals DeltaHB(i) decreases.

As shown , the expiration phase has a length **Texp,** starting at **T_ie** and ending at **T_ei**. The inspiration phase has a length **Tinsp,** starting at **T_ei** and ending at **T_ie**.

$$\text{The overall respiration period is denoted Tresp} = \text{Tinsp+Texp.}$$

**A** state of stress and/or relaxation of the user can be assessed as a function of synchronization index between the inspiration/expiration phases and the user heart beats.

The phase between the heart cycle and its modulation by the respiration can be calculated by standard signal processing methods of sampling and reconstruction, interpolation, or curve fitting, for instance of a cosine.

In an exemplary embodiment, the respiration cycle can be reconstructed from steps above, as a cosine-like respiration cycle, with null phase for instance at a time of switch between inspiration and expiration (namely **T_ie**) or at a time of switch between expiration and inspiration (namely **T_ei**). A possible method of reconstruction is a minimal least squares regression on the wave amplitudes **WA**(i) and heart beats **HB(i).**

At each the beginning of each respiration cycle, the phase difference of the cosine **DeltaPhi(j)** can be defined as a phase difference which separates the null phase of the respiration cycle **j** and the nearest heart beat HB(i).

The respiration cycles and the heart cycles are synchronous if **DeltaPhi(j)** is constant over several respiration cycles.

In particular, whenever the respiration cycles and the heart cycles are synchronous, this denotes a state of relaxation and well-being of the user U. At the contrary change of the phase **DeltaPhi(j)** with the cycle **j** denotes a state of stress.

Alternately, the modulation of the heart periods by the respiration can be reconstructed from the heart beats HB(i) obtained from the feet or the apexes Y of the IPG. As described above, the phase difference DeltaPhi(j) is calculated at the beginning of each respiration cycle.

According to a case illustrated at figure 9, **DeltaPhi** is constant, the user is thus relaxed. DeltaPhi is the same close to HB(2), HB(5) and HB(8)

Conversely, according to a case illustrated at figure 10, **DeltaPhi** is not constant, and in this case, the user U is subject to stress. More precisely it is apparent that **DeltaPhi-1at** HB(2) is rather small, **DeltaPhi-2** at HB(5) is larger and **DeltaPhi-3** at HB(8) is even larger.

It is also known that the strength of this amplitude and frequency modulation depends on the emotional state of the person. A level of relaxation or stress can be indicated therefrom to the user.

In summary, the synchronization index can be taken from a derivative over time of DeltaPhi; in other words the synchronization index is defined from an evolution over time of a phase difference **DeltaPhi,**

It is noted that the variability of the heart rate could be calculated with other fiducial points than **J,** for instance with the apexes **I,** or the average values of **JJ** or **II** intervals.

It is noted that the variability of the heart rate could also be calculated with fiducial points of the IPG, for instance the foot of the beat or its apex Y1-Y9.

According to a further aspect, the time intervals **DeltaHB(i)** between successive J apexes (or successive I apexes or successive Y apexes) are also modulated by the general state of fatigue of the person. This state can be determined with the help of a heart rate variability index denoted HRVI.

In particular example, beat time intervals DeltaHB(i) between successive heart beats are defined by the measured time intervals between the second positive apexes **J** of each heart beat of a couple of successive heart beats, from the ballistocardiogram signal 21.

In another example, time intervals between the successive apexes **Y** of the impedance plethysmography signal 22 are measured.

According to first possibility, such a heart rate variability index HRVI can be expressed by Max **[DeltaHB(i)] -** Min **[DeltaHB(i)],** where indicia i is ranging from 1 to **i0**, i0 being the number of monitor heart beats when the user is standing on the scale, i0 being at least 6.

According to another possibility, such a heart rate variability index HRVI can be expressed by the average over several complete respiration cycles of the differences Max **[DeltaHB(i)] -** Min **[DeltaHB(i)]** calculated over each respiration cycle (detected as explained above), namely where the index **i** ranges over the indicia of the heart beats in the given respiration cycle. Alternately, if only one complete respiration cycle is recorded, the heart rate variability index HRVI is expressed by **Max [DeltaHB(i)] - Min [DeltaHB(i)]** where the index **i** ranges over the heart beats of the complete respiration cycle.

According to another possibility, the heart rate variability index HRVI can be inferred from time parameters such as the Root Mean Squared of the Successive Differences (RMSSD) of **DeltaHB(i)** over several successive heart beats, as follows:

$$HRVI = \sqrt{\sum_{k1}^{k2-2} \frac{([DeltaHB(i+2) - DeltaHB(i+1)]^2}{k2 - k1 - 1}}$$

This estimate of the Heart Rate Variability index (HRVI) is stored on the servers and compared to previously recorded values. A level of the general state of fatigue can be given back to the user, this level being relative to the past state of fatigue that has been recorded. For instance, the feed back indicates to the user that he is more tired (or much more tired, or more rested, etc) than the previous day, or the previous week.

Advantageously, averaging over several measurements permits to smooth out the variability introduced to the different emotional states of the person during the measurements in order to get a value more representative of the general, mid-term state of fatigue of the user.

Advantageously, the user can be asked on at least one occasion to assess himself his state of fatigue and give that information via the smartphone application. This datum is stored on the server and used improve the precision of the feedback to the user.

According to a further aspect, illustrated at Figure 11, which is independent from the ballistocardiogram signal analysis, at each heart beat, at least a portion of the decreasing part of the impedance plethysmogram after the maximum can be analysed to assess the peripheral resistance of the cardiovascular system. More precisely, as explained above, the impedance plethysmogram is produced by the pulsatile volume of blood in the arteries which is caused by, and follows closely, the pulsatile blood pressure in the arteries. It is known that during diastole the blood pressure decays approximately according to an exponential as follows:

$$P(t) \approx P\max . e^{\left(\frac{-t}{RP.C}\right)}$$

**RP** is the peripheral resistance to blood flow as described above. Pmax is the height of point Y.
**C** is the artery's **compliance** which is a value is deduced from the measurement of the PWV, as seen above.

As illustrated in <u>figure 8</u>, the signal 22 obtained at the foot reflects the cut in the general relationship , with in particular :

$$P(t) = P\max . e^{\left(\frac{-t}{RP0.C}\right)}$$

**RP0** is the peripheral resistance that can be deducted from the shape of the impedance curve after the apex **60.** A fast decrease **61** in the impedance reflects a small (**RP0**.**C**) time constant; conversely, a slow decrease **64** in the impedance reflects a high (**RP0**.**C**) time constant. Therefore, the decrease rate of the curve **62,63** can be analyzed to retrieve the value of **RP0**.

Advantageously, as illustrated in <u>Figure 10</u>, this estimate of the peripheral resistance **RP0** can be combined to the estimate obtained from the Mean Arterial Pressure and Cardiac Output **(RP** = **MAP** / **CO, see above)** in order to calculate a more reliable value **RPP** of the peripheral resistance.

**Claims**

1. A **Method** to determine a **blood arterial pressure** of an individual user (U) in a system comprising a smartphone (2), a cuff pressure monitor device (6) and a personal electronic scale (1) having a top surface with conductive pads, the method comprising the steps of :

    S1- measure a first mean arterial pressure (**MAP1**) of the individual user (U) with the help of the cuff pressure monitor device, at a first instant (GT1),
    S2- determine a first arterial pulse wave velocity value (PWV1) of the individual user (U) standing on the personal electronic scale, at a second instant (GT2), temporally close to the first instant (GT1),

        /**a**/- acquiring weight variations, and extracting therefrom a ballistocardiogram (21) of the user's heart beat,
        /**b**/- acquiring impedance plethysmography signals across one of the foot of the user, and extracting therefrom a blood pulse signal (22) at the foot,
        /**c**/-calculating a time delay (DT) between the heart beat and the blood pulse signal arriving at the foot,
        /**d1**/ deducing therefrom a value of the arterial pulse wave velocity of the user,

    S3- determine a second arterial pulse wave velocity value (PWV2) in a similar manner as for step S2, at a third instant (GT3),
    S4- determine a second mean arterial pressure (**MAP2**) of the individual user (U) from the first mean blood pressure MAP1 and a function Fcorr of **PWV1** and **PWV2**, namely

$$\mathbf{MAP2 = MAP1 + Fcorr\ (PWV1, PWV2).}$$

2. The method of claim 1, wherein

    - a determination of an arterial pulse wave velocity can be performed each time the user (U) stands on the personal electronic scale (step **S2** and **S3**), for example on a daily basis, and
    - the measurement of a first mean blood pressure MAP1 of the individual user (U) with the cuff pressure monitor device (step S1) is performed at a lower frequency, for example once a month.

3. A **Method** to assess a **stroke volume** of the heart systolic contraction of an individual user (U) standing on a personal electronic scale (1), the method comprising :

/a/- acquire weight variations, and extracting therefrom a ballistocardiogram signal (21) reflecting the user's heart beats,

- identify, in the ballistocardiogram signal, for <u>at least one</u> heart beat **HB**(i) exhibiting a pulse-like wave signal **PW**(i), having a first and a second significant negative apexes **I,K** and a first and a second significant positive apexes **H,J,**
- identify, one or more characteristic value **WCV** from at least two of the first and second positive and negative apexes, H,I,J,K,
- assess a user's stroke volume **SV,** as a mathematical function of the one or more characteristic value **WCV.**

4. The method of claim 3, wherein the characteristic value WCV is given by :

$$\mathbf{WCV} = \int_{H}^{Q} |PW(i)(t)| dt,$$

Q being either I or J, and SV is inferred from WCV.

5. The method of claim 3, wherein the one or more characteristic value WCV comprise :

- a first amplitude **A1**, measured from the first positive apex **H** to the first negative apex **I,** a second amplitude **A2**, measured from the first negative apex **I** to the second positive apex **J,** a third amplitude **A3**, measured from the second positive apex **J** to the second negative apex **K,**

wherein_the stroke volume is given by **SV = G (A1,A2,A3)**, G being a linking function with predefined coefficients.

6. The method of claim 5, wherein the linking function **G** can be expressed by :

$$\mathbf{G} = \mathrm{K} \times \sqrt{\alpha 1 A1 + \alpha 2 A2 + \alpha 3 A3} \times (\mathrm{HR})^{\mathrm{BR}}$$

where K, $\alpha 1$, $\alpha 2$, $\alpha 3$ and BR are either predefined coefficients and HR is the user's heart rate.

7. The method according to claim 1 and to claim 3, further comprising:

- determine user's Heart Rate (HR) and cardiac output CO by CO = HR x SV
- determine a user's cardiovascular parameter known as peripheral resistance **RP**, by dividing the mean arterial pressure by the Cardiac output, namely RP = MAP1/ CO, or RP = MAP2/ CO.

8. A **Method** to assess a **state of stress and/or relaxation** of an individual user (U) standing on a personal electronic scale (1), the method comprising :

/**a**/- acquire weight variations, and extracting therefrom a ballistocardiogram signal (21) reflecting the user's heart beats,

/**PA1**/- identify, in the ballistocardiogram signal (21), <u>for each</u> of a plurality of consecutive heart beats HB(i), a pulse wave PW(i),
/**PA2**/- measure, for each pulse wave PW(i), at least one characteristic amplitude WA(i) of the pulse wave PW(i),

- measure, for each couple of consecutive heart beats, beat time intervals DeltaHB(i) between successive heart beats, from the ballistocardiogram signal (21) <u>or</u> from an impedance plethysmography signal (22) measured at the user's foot,

/**PR1**/- determine a user expiration phase whenever characteristic amplitude WA(i) decreases and/or beat time intervals DeltaHB(i) increases,
/**PR2**/- determine a user inspiration phase whenever characteristic amplitude WA(i) increases and/or beat time intervals DeltaHB(i) decreases,

- assess a state of stress and/or relaxation of the user, as a function of synchronization index between the inspiration/expiration phases and the user heart beats.

9. The method of claim 8, further comprising :

/**PR3**/ - reconstruct, from steps /PR1/ and /PR2/, a respiration cycle as a cosine-like function of time, with null phase at a time of switch between inspiration and expiration (T_ie) or at a time of switch between expiration and inspiration (T_ei),

wherein the synchronization index is defined from an evolution over time of a phase difference DeltaPhi, which separates the null phase of the respiration cycle and the nearest heart beat HB(i).

10. The method of claim 8, wherein the characteristic amplitude **WA**(i) of the pulse wave **PW**(i) is defined by the amplitude $A_{JK}i$, measured from the second positive apex **Ji** to the second negative apex **Ki** or the amplitude $A_{IJ}i$ measured from the first negative apex Ii to the second positive apex **Ji.**

11. A **Method** to assess a **state of fatigue** of an individual user (U) standing on a personal electronic scale (1), the method comprising :

- measure, for each couple of consecutive heart beats, beat time intervals **DeltaHB(i)** between successive heart beats, extracted from a ballistocardiogram signal (21) or from an impedance plethysmography signal (22) measured at the user's foot,
- determine a heart rate variability index **HRVI,** over at least six successive heart beats.

12. The method of claim 11, wherein the heart rate variability index **HRVI** can be expressed by :

$$Max\ [\mathbf{DeltaHB(i)}] - Min\ [\mathbf{DeltaHB(i)}],\ \text{where indicia } \mathbf{i} \text{ is ranging from 1 to } \mathbf{i0},\ \text{i0 being the}$$

where indicia i is ranging from 1 to **i0**, i0 being the number of monitored heart beats when the user is standing on the scale, **i0** being at least 6.

13. The method of claim 12, wherein **i0** is defined such that at least one complete respiration cycle is recorded during **i0** heart beats, preferably more one complete respiration cycle are recorded, whereby the respiration cycle is retrieved by the following steps :

/**PA1**/- identify, in the ballistocardiogram signal (21), for each of a plurality of consecutive heart beats HB(i), a pulse wave **PW**(i),
/**PA2**/- measure, for each pulse wave **PW**(i), at least one characteristic amplitude **WA**(i) of the pulse wave **PW**(i),
/**PR1**/- determine a user expiration phase whenever characteristic amplitude **WA**(i) decreases and/or beat time intervals DeltaHB(i) increases,
/**PR2**/- determine a user inspiration phase whenever characteristic amplitude WA(i) increases and/or beat time intervals DeltaHB(i) decreases.

14. The method of claim 11, wherein the heart rate variability index **HRVI** can be expressed by :

$$HRVI = \sqrt{\sum_{k1}^{k2-2} \frac{([DeltaHB(i+2) - DeltaHB(i+1)]^2}{k2 - k1 - 1}}$$

**FIG. 1**

**FIG. 2**

**FIG. 3**

**FIG. 4**

# FIG. 5

# FIG. 6

**FIG. 7**

**FIG. 8**

## FIG. 9

## FIG. 10

# FIG. 11

```
┌──────────────┐              ┌──────────────────┐
│   Ballisto   │              │    Impedance     │
│  Cardiogram  │              │ Plethysmography  │
│     BCG      │              │       IPG        │
└──────────────┘              │    (at foot)     │
                              └──────────────────┘
```

Stroke Volume

Heart Rate **HR**

Pulse Wave Velocity (Arterial Stiffness **AS**), Compliance **C**

**CO** = HR x SV

Cuff Blood Pressure Monitor

Mean Arterial Pressure **MAP**

Peripheral Resistance **RP**

**RP0**

**RPP**

# FIG. 12

Cuff S1

MAP1

PWV1

S2

PWVi

S3

PWV2

MAP2

S4

Cuff Recalibraion S1'

GT1

GT2

GT3

Time

**EP 3 095 380 A2**

**Patent documents cited in the description**

- US 8639226 B, Withings
- WO 2014106716 A, Withings
- US 2013310700 A, Stanford
- US 20140309541 A